# EUROPÄISCHE PATENTANMELDUNG

(11) **EP 0 651 981 A1**
(43) Veröffentlichungstag der Anmeldung: **10.05.1995**
(21) Anmeldenummer: 94117754.5
(22) Anmeldetag: 10.11.1994
(51) Int. Cl.: A61F 9/00, A61B 3/00, G02B 6/32, G02B 6/42

(54) **Ophthalmoskopische Beleuchtungssonde**

(30) Priorität: 10.11.1993 CH 3375/93
(71) Anmelder: VOLPI AG, CH-8952 Schlieren (CH)
(72) Erfinder: Jenny, Reinhard, CH-5400 Ennetbaden (CH)
(74) Vertreter: Ritscher, Thomas, Dr.

(57) **Zusammenfassung**

Die Beleuchtungssonde ist insbesondere zum seitlichen Einstechen in den Augapfel bei der Ophthalmoskopie vorgesehen. Sie enthält eine Lichtleitfaser (14), deren austrittsseitiges Ende mit einer Einrichtung (15, 12) zum Vergrössern des Raumwinkels des abgestrahlten Lichts und damit der beleuchteten Fläche geeignet ist. Ausführungsformen dieser Einrichtung sind beispielsweise ein am Faserende angeordnetes oder in das Faserende eingearbeitetes Frenel-linsenartiges Element (15) oder eine in das Faserende eingearbeitete zur Faserachse symmetrische konkave Ausnehmung (12). Die Beleuchtungssonde kann mit einem Bildleiter kombiniert werden.

## Beschreibung

Die vorliegende Erfindung betrifft eine ophthalmoskopische Beleuchtungssonde nach dem Oberbegriff des Anspruchs 1 und deren Verwendung.

In der modernen Augenheilkunde werden stets mehr mikroskopische Untersuchungen und mikrochirurgische Operationen vorgenommen. Die klassischen in der Augenheilkunde verwendeten Lichtquellen, also Spaltlampe, Augenspiegel etc., eignen sich für diese neuen Diagnose- und Therapieanwendungen nicht mehr. Es werden deshalb speziell entwickelte endoskopische Beleuchtungsvorrichtungen eingesetzt, die mit entsprechenden mikroskopischen oder elektrooptischen Bildsystemen zusammenwirken.

So ist beispielsweise aus der EP-512'592 ein Laser-Video-Endoskop bekannt, bei welchem das erforderliche Licht über ein Lichtfaserbündel zu einer in einem Handstück befestigten Sonde geleitet wird. Diese Sonde weist auch ein geordnetes Bildleiterbündel und eine Laserlicht-Monofaser auf. Das in dieser EP-512'592 beschriebene Beleuchtungssystem weist ein weites Bildfeld auf und ermöglicht damit dem behandelnden Arzt gleichzeitig verschiedene Gewebezonen zu erkennen, wodurch die Orientierung und das Arbeiten im Augeninnern wesentlich erleichtert wird. Dieses Endoskop eignet sich speziell zur Photokoagulation der Retina, der Cornea oder jeder anderen Hautschicht des Auges.

Dabei wird von der thermischen Wechselwirkung zwischen Laserlicht und Gewebe gezielt Gebrauch gemacht. Zur Photokoagulation werden heute beispielsweise Argonlaser mit Wellenlängen von 457nm bis 524nm, Kryptonlaser mit Wellenlängen von 647,1nm (rot) 568,2nm (gelb) und 530,8nm (grün) oder CO₂-Laser mit 10,6µm Wellenlänge eingesetzt. Die für diese Operation benötigte Leistungsdichte des Laserlichtes beträgt etwa 1-100 Watt/cm² und muss mit der Laserpulsdauer, ca. 1/100 Sek. bis über 1 Min., abgestimmt sein. Die damit erzeugten lokalen Temperaturerhöhungen sind für den Erfolg dieser Art Operationen von besonderer Bedeutung. Insbesondere werden Temperaturen von ca. 162° Celsius für die gewünschte Koagulation benötigt, während Temperaturen von ca. 180° Celsius bereits zu Collagen-Denaturierung und Carbonisierung führen. Damit wird deutlich, dass die mit der Beleuchtung für das Beobachtungssystem eingestrahlte Energie bei dieser Art Operationen mitzuberücksichtigen ist.

Leider zeigt sich bei dieser Art endoskopischer Abbildungssysteme, dass die mit dem Faserbündel beleuchtete Fläche eine rasterartige Hell-Dunkel-Struktur aufweist, die erzeugten Bilder kontrastarm sind und keine befriedigende Bildqualität aufweisen. Deutlich wird dieser Mangel insbesondere beim Auftreten von starken Reflexen im Bildfeld, welche das ohnehin kontrastarme Bild stark überstrahlen. Dieser Effekt erweist sich in der Praxis als äusserst bedeutsam, da gerade in der Mikrochirugie sowohl die zu behandelnden Gewebezonen als auch die für die Operation benötigten metallischen Instrumente zu beobachten sind. Es wurde deshalb versucht, möglichst viel Licht in die lichtleitenden Fasern einzukoppeln, um beim Unterdrücken der Blendung ein genügend kontrastreiches Bild zu erhalten. Diese Lösung erweist sich jedoch als unpraktikabel, da mit der stärkeren Beleuchtung photochemische Reaktionen im Gewebe des Auges induziert werden, die unter dem Begriff "Lichttoxizität" zusammengefasst werden. Diese Lichttoxizität umfasst zytotoxische und mutagene Nebenwirkungen in den Zellen, die erst nach Jahren beispielsweise eine mehr oder weniger starke Trübung der Hornhaut verursachen können. Lichttoxizität ist heute Gegenstand von intensiven medizinischen Untersuchungen.

Es ist deshalb Aufgabe der vorliegenden Erfindung eine Beleuchtungssonde für ophthalmoskopische Abbildungssyteme zu schaffen, welche die Nachteile der oben erwähnten Vorrichtungen überwindet. Insbesondere soll ohne aufwendige Massnahmen eine Beleuchtungssonde geschaffen werden, mit welcher ohne Bildqualitätseinbusse die erwähnte Lichttoxizität vermieden und die Lichtverteilung homogenisiert wird.

Erfindungsgemäss wird diese Aufgabe durch eine Beleuchtungssonde mit den Merkmalen des Anspruchs 1 gelöst und insbesondere durch eine Beleuchtungssonde, an deren lichtaustrittseitigen Ende Mittel vorgesehen sind, welche den Raumwinkel des abgestrahlten Lichtstroms vergrössern und homogenisieren.

Weitere bevorzugte Ausführungsformen ergeben sich aus den Merkmalen der Unteransprüche.

Die durch die erfindungsgemässe Beleuchtungssonde erzielten Vorteile sind für den Fachmann unmittelbar deutlich. Insbesondere können dadurch die erwähnten lichttoxischen Nebenwirkungen vermieden werden, ohne die Beleuchtungsstärke vermindern zu müssen. Dadurch kann die Bildqualität, und insbesondere die Kontraststärke trotz Gesichtsfeldvergrösserung beibehalten werden.

Die vorliegende Erfindung soll im folgenden anhand bevorzugter Ausführungsbeispiele und mit Hilfe der Figuren näher erläutert werden.
- Fig. 1:: zeigt ein Lichtleistungsdichte-Beleuchtunszeit-Diagramm;
- Fig. 2:: zeigt die Verwendung einer erfindungsgemässen Sonde am Auge in schematischer Weise;
- Fig. 3:: zeigt eine erste Ausführungsform der erfindungsgemässen Sonde mit einer kegelförmigen Spitze;
- Fig. 4:: zeigt eine weitere Ausführungsform der erfindungsgemässen Sonde mit einer konkaven Rotationsfläche;
- Fig. 5:: zeigt eine erfindungsgemässe Sonde mit einem Fresnel-Element;
- Fig. 6:: zeigt eine erfindungsgemässe Ausführungsform für eine endoskopische Sonde.
- Fig. 7:: zeigt eine Weiterbildung der erfindungsgemässen Sonde mit einer Beleuchtungsfaser, die unmittelbar vor ihrem austrittsseitigen Ende einen Hohlraum aufweist.

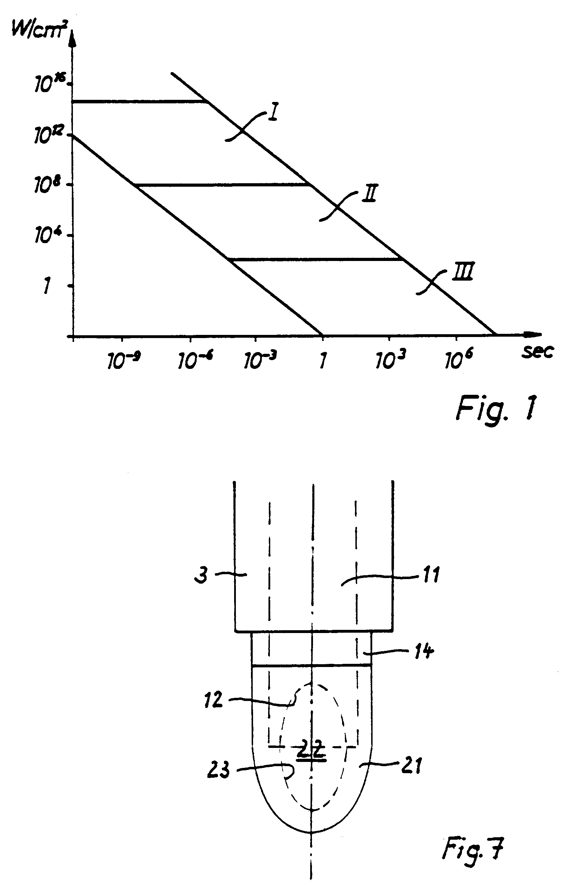

Die physiologischen Wirkungen von Lichtstrahlen auf die verschiedenen Gewebeschichten sind in Fig. 1 schematisch dargestellt. Grundsätzlich können diese in drei Wirkungs-Bereiche I,II,III, eingeteilt werden.

In einem ersten Bereich I, in welchem durch hochenergetische Lichtimpulse im Picosekunden-Bereich ein lokales Plasma erzeugt werden kann, kann das belichtete Gewebe vergast und/oder verflüssigt werden. Damit können, wie das beispielsweise bei Operationen des Grauen Stars notwendig ist, trübgewordene Gewebeschichten oder die gesamte Augenlinse abgetrennt werden, ohne das Auge selbst öffnen zu müssen.

Im nächsten Bereich II führt die dem Gewebe zugeführte Lichtenergie zur Denaturierung der Proteine und wirkt dadurch koagulierend. Behandlungen mit Licht in diesem Bereich Oumfassen im wesentlichen die "Verschweissung" einer sich ablösenden Netzhaut mit der darunterliegenden Aderhaut. Insbesondere wird Licht mit einer Wellenlänge im blau-grünen Wellenlängenbereich im Hämoglobin des Blutes stark absorbiert und eignet sich deshalb besonders für die Blutstillung bei Blutgefässen. Dabei werden Beleuchtungszeiten von 0,1 bis 1 sec. und Leistungen von 0,1 bis 1 Watt verwendet. Behandlungen mit anderen Wellenlängen beispielsweise 568nm (gelb) oder 647nm (rot) erweisen sich für die Koagulation von anderen Zellverbänden, d.h. für das Verbinden von anders zusammengesetzten Hautschichten als besonders geeignet.

Im dritten Bereich III (Milliwatt-Bereich) finden photochemische Reaktionen statt, wie sie für das normale Funktionieren des Auges im wesentlichen bekannt sind. Obwohl diese photochemischen Reaktionen im allgemeinen als reversibel betrachtet werden können, hat es sich nun gezeigt, dass bei längerer Beleuchtung der photoempfindlichen Hautschichten mit Licht im Milliwatt-Bereich, in diesen Schichten irreversible Veränderungen erzeugt werden können. Insbesondere konnten bei verschiedenen Patienten nach mehreren Jahren zytotoxische und mutagene Nebenwirkungen festgestellt werden. Eine mögliche Ursache dieser Phototoxizität wird in der für die Diagnose und die Operation notwendigen Langzeit-Beleuchtung gesehen. Hier will die vorliegende Erfindung Abhilfe schaffen.

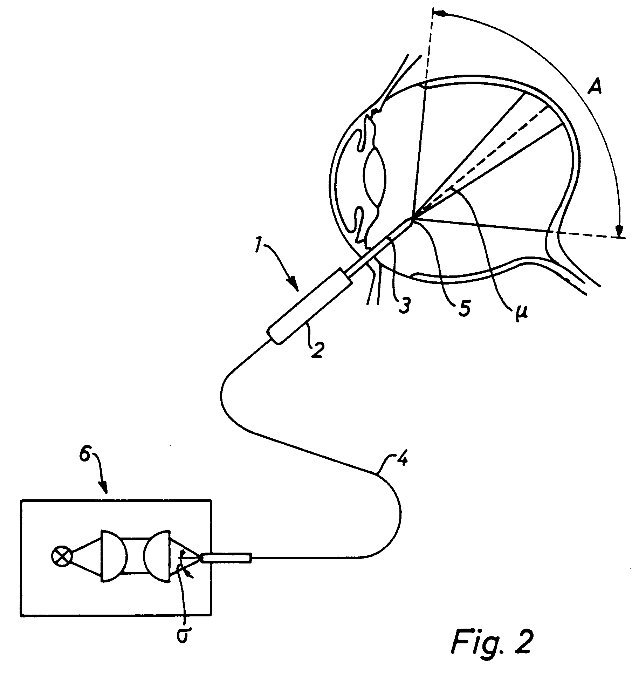

Fig. 2 zeigt die Verwendung einer erfindungsgemässen Beleuchtungssonde 1 in schematischer Weise. Diese weist in einer bevorzugten Ausführungsform ein Handstück 2 mit einer Kanüle 3 auf, in welcher ein Lichtleiter 4 liegt, der Licht von einer Beleuchtungseinheit 6 bis in die Spitze 5 der Beleuchtungssonde 1 leiten kann. Die Beleuchtungseinheit 6 kann auch Teil eines Mikroskopes sein, das dem behandelnden Arzt zur Beobachtung dient. Der Öffnungswinkel 2σ des auf den Lichtleiter 4 auftreffenden Lichtbündels beträgt in der Regel 85°, um sicherzustellen, dass Eintrittsaperturen von 24° bis 83°, wie sie durch die Materialien der verschiedenen Lichtleitertypen vorgegeben sind, vollständig im eingestrahlten Lichtkegel liegen. Die Abstrahlcharakteristik des Lichtleiters 4 hängt also wesentlich von der Eintrittsapertur des Lichtleiters resp. vom verwendeten Fasermaterial ab. Insbesondere nimmt die praktisch nutzbare Apertur u wegen der winkelabhängigen inneren Verluste mit wachsender Länge des Lichtleiters 4 ab und verringert dadurch das nutzbare Gesichtsfeld A.

Eine weitere Verringerung der nutzbaren Apertur u wird dadurch erzeugt, dass mit der Sonde im Augeninnern, d.h. in dem mit Kammmerwasser gefüllten Glaskörper des Auges gearbeitet wird. Dadurch wird die numerische Apertur d.h. der Öffnungswinkel des Austrittskegels wesentlich verkleinert und entsteht auf der zu beobachtenden Fläche ein stark leuchtender Lichtfleck.

Erfindungsgemäss wird unter Beibehaltung des für eine hohe Bildqualität erforderlichen Lichtstroms, die Beleuchtungsstärke durch eine Vergrösserung der Apertur des austretenden Lichtstrahls reduziert. Dies kann erfindungsgemäss durch eine spezielle Formgebung des lichtaustrittseitigen Endes des Lichtleiters 4 erzielt werden.

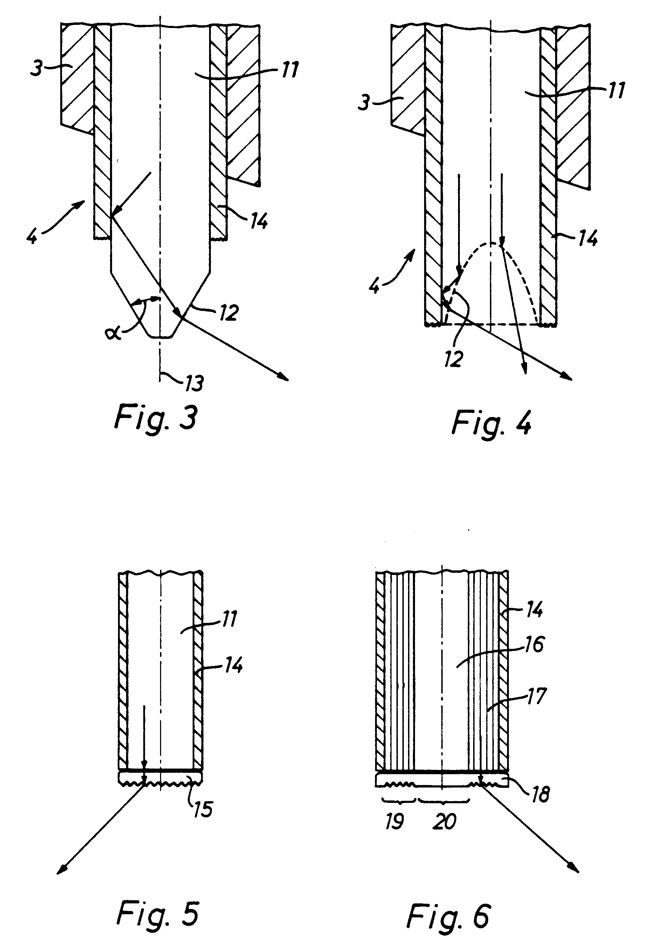

Fig. 3 zeigt die Spitze 5 einer erfindungsgemässen Sonde 1 mit einer Monofaser als Lichtleiter 4, deren Kern 11 eine konisch zusammenlaufende Körperfläche 12 aufweist. Der Neigungswinkel α kann nun derart gewählt werden, dass durch die erzielte Vergrösserung des Bildfeldes A die Bestrahlungsstärke derart reduziert wird, dass die phototoxischen Effekte nicht mehr auftreten. In einer erprobten Ausführungsform wurde die Körperfläche 12 um einen Winkel von ca. 15° gegen die optische Achse 13 geneigt. Dabei wurde eine Monofaser von 750µm Durchmesser, d.h. mit einem Kern 11 von ca. 730µm Durchmesser und einem ca. 10µm dicken Cladding, verwendet. Die Mantelfläche 12 wurde bei dieser Ausführung glatt poliert, um Reflexions-verluste des Lichtstroms an der Austrittfläche 12 zu vermeiden. Vorteilhafterweise wird der Kern 11 vor der Bearbeitung vom Cladding 14 befreit.

Eine andere erprobte Ausführungsform ist in Fig. 4 dargestellt. Wiederum wurde dafür eine Monofaser 4 aus PMMA mit ca. 750µm Durchmesser von ihrem Cladding 14 befreit. Die rotationsymmetrische Körperfläche 12 begrenzt hier als Ausnehmung das Faserende innenseitig. Dabei hat die Körperfläche 12 einfacherweise die Form einer Kegelfläche, kann aber vorteilhafterweise eine gekrümmte Rotationsfläche sein. Wesentlich ist lediglich die durch die Formgebung erzielte Aufweitung des Austrittstrahls und dessen gleichmässige räumliche Lichtstromverteilung. Um den Lichtverlust möglichst gering zu halten ist die Körperfläche 12 als glatte Fläche geformt, wobei die Ausbildung mit leichten Ondulationen auf dieser Fläche durchaus im Rahmen des normalen technischen Handelns liegen.

Weitere geometrische Ausführungsformen der erfindungsgemässen Sonde lassen sich aus dem oben gesagten für den Fachmann ohne weiteres ableiten. So kann beispielsweise statt einer Monofaser ein Bündel von 0.5µm dünnen Fasern verwendet werden, die an ihrem Ende miteinander verschmolzen sind. Denkbar ist auch eine rotationsymmetrische Körperfläche 12 in Form einer Einschnürung.

Die erfindungsgemässe Vergrösserung der Apertur kann aber auch durch mikrooptische Bauelemente realisiert werden. Figur 5 zeigt eine Monofaser 11 mit einem Cladding 14 und einer planen Stirnfläche auf welche ein Fresnel-linsenartiges Element 15 aufgebracht ist. Es versteht sich, dass die Fresnel-artige Struktur direkt in die Monofaser eingearbeitet sein kann. Dieses kann eingeprägt, eingeätzt oder durch andere Behandlungen in die Stirnseite integriert sein. Figur 6 zeigt das Ende einer endoskopischen Sonde mit einem Bildleiter 16 um welchen Beleuchtungsfasern 17 angeordnet sind. Wiederum ist stirnseitig ein mikrooptisches Element 18 angebracht welches jedoch nur auf der mit den Beleuchtungsfasern zusammenwirkenden Flächenteil 19 mit einer Fresnel-artigen Struktur versehen ist. Es versteht sich für den Fachmann, dass der mit dem Bildleiter 16 zusammenwirkende Flächenteil 20 dieses Elementes 18 als Sammellinse ausgebildet sein kann.

In Figur 7 ist eine Weiterentwicklung der Ausführungsform gemäss der Figur 4 gezeigt. Diese weiterentwickelte Ausführungsform weist am lichtaustrittseitigen Ende der Beleuchtungsfaser 11 eine Kappe 21 auf, die über das Cladding 14 bis zur Kanüle 3 geschoben ist. Die Kappe besteht vorzugsweise aus dem gleichen Material wie die Beleuchtungsfaser und enthält eine Ausnehmung 23, die die Körperfläche 12 zu einem Hohlraum 22 ergänzt. Um die Wirkung des Hohlraums 22 zu beeinflussen, weisen Körperfläche 12 und Ausnehmung 23 vorzugsweise nicht die gleiche Krümmung auf. Bei einer praktisch erprobten Ausführungsform beträgt die Länge der Kappe etwa 4 mm und die Länge des Hohlraums etwa 2 mm.

Es ist natürlich auch möglich bei der Herstellung der Beleuchtungsfaser, in diese, zum Erzeugen des Kohlraums, mit einer seitlich angesetzten Mikrosonde Luft, ein inertes Gas oder eine Flüssigkeit einzupressen. Der Düsenkanal schliesst sich nach dem Ausziehen der Mikrodüse durch Fliessen oder Kriechen des Fasermaterials von selbst. Die Wirkung des Hohlraums kann noch verstärkt werden, wenn dieser mit einem Material gefüllt wird, dessen Brechungsindex vom dem des Fasermaterials stark abweicht.

Es versteht sich, dass die Lichtaustrittsfläche einer Beleuchungssonde gemäss der Figur 7 zur zusätzlichen Vergrösserung des Lichtaustrittswinkels auch mit einem mikrooptischen Bauelement, wie es beispielsweise in Figur 5 gezeigt ist, versehen werden kann.

Weitere Anwendungen der erfindungsgemässen ophthalmoskopischen Beleuchtungssonde liegen im Bereich des Fachmännischen. Insbesondere kann diese Beleuchtungssonde mit medizintechnischen Instrumenten kombiniert werden.

## Patentansprüche

1. Verfahren zur Beleuchtung eines Operationsfeldes bei ophthalmischen Operationen mit einer ophthalmoskopischen Beleuchtungssonde (1), dadurch gekennzeichnet, dass der Raumwinkel des von der Beleuchtungssonde (1) abgestrahlten Lichtstromes so vergrössert wird, dass die Lichtstärke des abgestrahlten Lichtstromes unterhalb der für irreversible fotochemische Reaktionen kritische Schwelle zu liegen kommt.

2. Verfahren nach Anspruch 1, dadurch gekennzeichnet, dass der Raumwinkel des abgestrahlten Lichtstromes durch Vergrössern der Austrittsapertur der Beleuchtungssonde (1) vergrössert wird.

3. Verfahren nach Anspruch 1, dadurch gekennzeichnet, dass die Lichtstärke des abgestrahlten Lichtstromes für alle Abstrahlungsrichtungen innerhalb des nutzbaren Gesichtsfeldes (A) konstant ist.

4. Ophthalmoskopische Beleuchtungssonde zur Durchführung des Verfahrens nach Anspruch 1 mit einem Lichtleiter (4), dadurch gekennzeichnet, dass am lichtaustrittsseitigen Ende des Lichtleiters (4) optische Mittel vorgesehen sind, welche die Austrittsapertur des Lichtleiters (4) vergrössern.

5. Beleuchtungssonde nach Anspruch 4, dadurch gekennzeichnet, dass der Lichtleiter (4) an seinem lichtaustrittsseitigen Ende eine gegenüber seiner optischen Achse (13) rotationssymmetrische und entlang dieser optischen Achse (13) unterschiedlich beabstandete Körperfläche (12) aufweist.

6. Beleuchtungssonde nach Anspruch 4, dadurch gekennzeichnet, dass die rotationssymmetrische Körperfläche (12) den Lichtleiter (4) aussenseitig begrenzt.

7. Beleuchtungssonde nach Anspruch 4, dadurch gekennzeichnet, dass die rotationssymmetrische Körperfläche (12) den Lichtleiter (4) innenseitig begrenzt.

8. Beleuchtungssonde nach Anspruch 7, dadurch gekennzeichnet, dass eine zum Aufsetzen auf das lichtaustrittsseitige Ende der Lichtleitfaser (11) geeignete Kappe (21) vorgesehen ist, die eine Ausnehmung (23) aufweist, welche mit der den Lichtleiter (4) innenseitig begrenzenden Körperfläche (12) der Faser einen Hohlraum (22) bildet.

9. Beleuchtungssonde nach Anspruch 4, dadurch gekennzeichnet, dass die Lichtleitfaser (11) dem lichtaustrittsseitigen Ende benachbart einen Hohlraum (22) aufweist, der mit Luft, einem inerten Gas oder einer Flüssigkeit gefüllt ist.

10. Beleuchtungssonde nach einem der Ansprüche 4 bis 9, dadurch gekennzeichnet, dass am lichtaustrittseitigen Ende des Lichtleiters (4) stirnseitig ein mikrooptisches Element vorgesehen ist.

11. Beleuchtungssonde nach Anspruch 10, dadurch gekennzeichnet, dass das mikrooptische Element als integrierte Struktur ausgebildet ist.

12. Beleuchtungssonde nach einem der Ansprüche 10 oder 11, dadurch gekennzeichnet, dass das mikrooptische Element ein Fresnel-linsenartiges Element ist.

13. Beleuchtungssonde nach einem der Ansprüche 4 bis 12, dadurch gekennzeichnet, dass der Lichtleiter (4) an seinem lichtaustrittseitigen Ende aus einem Faserbündel besteht.

14. Beleuchtungssonde nach einem der Ansprüche 4 bis 12, dadurch gekennzeichnet, dass der Lichtleiter (4) an seinem lichtaustrittseitigen Ende aus einer Monofaser besteht.

15. Beleuchtungssonde nach einem der Ansprüche 13 oder 14, dadurch gekennzeichnet, dass der Lichtleiter (4) durch eine Kanüle (3) geführt ist.
